Europäisches Patentamt

European Patent Office

Office européen des brevets

(11) Numéro de publication: **0 077 277**
A1

# (12) DEMANDE DE BREVET EUROPEEN

(21) Numéro de dépôt: **82401886.5**

(51) Int. Cl.³: **A 61 B 17/12**

(22) Date de dépôt: **13.10.82**

(30) Priorité: **14.10.81 FR 8119348**
**12.02.82 FR 8202366**

(71) Demandeur: **NOMEL, Tour Franklin Quartier Boieldieu, F-92081 Puteaux-La Défense (FR)**

(43) Date de publication de la demande: **20.04.83**
**Bulletin 83/16**

(72) Inventeur: **Hrouda, Georges, 42, rue du Cherche-Midi, F-75006 Paris (FR)**

(84) Etats contractants désignés: **AT BE CH DE GB IT LI LU NL SE**

(74) Mandataire: **Beauchamps, Georges et al, Cabinet Z.Weinstein 20, avenue de Friedland, F-75008 Paris (FR)**

(54) **Procédé et dispositif d'application de pinces hémostatiques et pinces hémostatiques appliquées par ce dispositif.**

(57) La présente invention concerne un procédé et un dispositif d'application de pinces hémostatiques.

Le dispositif d'application (20) de pinces hémostatiques (2) est en forme de pince comprenant deux bras (21, 22) reliés de manière pivotante et actionnant deux mâchoires (24, 25) dont l'une (24) comprend deux éléments de mâchoire (29, 30) agencés pour être appliqués successivement sur l'autre mâchoire (25) de manière à successivement former une boucle avec recouvrement des extrémités de la pince hémostatique (2) et écraser ladite boucle ainsi formée jusqu'à obturation du conduit souple.

L'invention s'applique notamment dans le domaine médical.

Procédé et dispositif d'application de pinces hémostatiques et pinces hémostatiques appliquées par ce dispositif

La présente invention concerne un procédé d'application
de pinces hémostatiques pour obturer un conduit à paroi
souple et un dispositif d'application de ces pinces sur
ce conduit et des pinces hémostatiques.

Elles visent plus particulièrement un dispositif d'application de pinces hémostatiques pour la ligature
rapide de vaisseaux sanguins nécessitée en chirurgie,
lors de l'ablation d'un organe par exemple, ce dispositif d'application étant d'une utilisation aisée et
sûre pour le chirurgien.

L'utilisation de pinces hémostatiques pour serrer et
obturer des vaisseaux sanguins pendant une opération
chirurgicale est bien connue dans la pratique, et de nombreux dispositifs d'application de pinces et des pinces
hémostatiques ont fait l'objet de nombreux brevets antérieurs.

Les dispositifs d'application de pinces hémostatiques
connus permettent simplement de rapprocher les branches
d'une pince hémostatique, ce rapprochement étant limité
par la grosseur du vaisseau à obturer ou de l'épaisseur
de chair adhérente à la paroi de ce vaisseau. Ainsi, il
est impossible avec le dispositif d'application connu de
verrouiller positivement et de manière sûre les extrémités des pinces hémostatiques pour éviter l'ouverture
desdites pinces au cours de l'opération chirurgicale.

La présente invention a pour but de remédier à tous ces
inconvénients en proposant un dispositif d'application
de pinces permettant au chirurgien de prélever dans un dispositif distributeur de pinces ou un dispositif support, les pinces une par une par simple positionnement du dispositif d'application dans ledit distributeur, et d'appliquer la pince hémostatique ainsi prélevée autour d'un vaisseau en réalisant une obturation sûre dudit vaisseau avec un verrouillage positif des extrémités de la pince hémostatique.

A cet effet, l'invention a pour objet un procédé d'application de pinces hémostatiques consistant à retirer une pince hémostatique, par exemple, d'un dispositif distributeur de pinces hémostatiques, au moyen d'un dispositif d'application de pinces, de loger un conduit souple à obturer tel qu'un vaisseau sanguin, entre les deux branches de la pince hémostatique ainsi prélevée et à obturer ledit conduit par fermeture de ladite pince.

Selon l'invention, on réalise la fermeture de ladite pince en effectuant successivement un rapprochement des extrémités de la pince pour former une boucle, un recouvrement desdites extrémités pour verrouiller ladite pince et enfin un écrasement de la boucle ainsi formée jusqu'à obturation du conduit souple.

Mais pour mettre en œuvre ce procédé, la présente invention vise également un dispositif d'application de pinces hémostatiques, ledit dispositif d'application étant en forme de pince comprenant deux bras reliés de manière pivotante et actionnant deux mâchoires, lesdits bras comportant à leur extrémité libre des moyens de préhension généralement en forme de bague adaptée à la forme des doigts d'une main.

Selon l'invention, ce dispositif se caractérise en ce que l'une des mâchoires comprend deux éléments agencés pour former une boucle avec recouvrement des extrémités de la pince hémostatique, et un second élément pour écraser la boucle ainsi formée jusqu'à obturation complète du conduit souple, ou vaisseau sanguin.

Selon un premier mode de réalisation de l'invention, le premier élément précité est relié à un organe élastiquement déformable, ledit organe étant relié de manière pivotante avec les deux bras précités du dispositif d'application, le pivotement et la déformation dudit organe étant commandés par ces deux bras. Le second élément est disposé dans une ouverture centrale dudit premier élément, ledit second élément étant relié au moins temporairement à l'un des bras du dispositif. Selon un mode de réalisation préféré de l'invention, ce second élément vient de matière avec un bras du dispositif d'application de pinces.

Avantageusement, au moins une des mâchoires comprend sur sa surface interne une rainure longitudinale formant moyen de réception de la pince hémostatique , permettant un positionnement correct de celle-ci entre les deux mâchoires du dispositif d'application de pinces.

Dans le premier mode de réalisation préféré de l'invention, et notamment pour l'application de pinces hémostatiques comportant une branche à extrémité recourbée, la mâchoire sur laquelle sont appliqués les deux éléments précités comprend une extrémité recourbée formant moyen de réception de ladite pince pour maintenir et guider au moins une partie de ladite extrémité recourbée de la pince hémostatique, tandis que l'extrémité du premier élément comprend un élément déformant susceptible d'agir sur ladite extrémité recourbée de la pince lors d'un mouvement de fermeture des mâchoires, pour réaliser le recouvrement des deux extrémités de la pince hémostatique et le verrouillage positif de celle-ci.

Avantageusement, cet élément déformant comprend à sa surface inférieure une gorge dont le fond définit une courbe convexe pour guider et plier l'extrémité recourbée de la pince hémostatique au-dessus de l'extrémité de l'autre branche de ladite pince, et dont les parois de la gorge forment les surfaces de guidage pour éviter tout déplacement latéral de ladite pince hémostatique.

Selon un second mode de réalisation de l'invention, le premier bras du dispositif d'application de pinces hémostatiques comprend un premier élément de bras relié audit premier élément de mâchoire, et un second élément de bras relié audit second élément de mâchoire.

Ainsi, avec ce dispositif, le chirurgien forme une boucle verrouillée autour du conduit souple par rapprochement du second bras et du premier élément de bras, puis par actionnement du second élément de bras le chirurgien déforme la boucle ainsi formée par rapprochement du second élément de mâchoire de la seconde mâchoire, par application d'une force correspondant sensiblement à la résistance à la déformation du vaisseau sanguin à obturer.

Avantageusement, ce dispositif comprend un élément formant ressort de rappel disposé entre les premier et second éléments de bras précités pour maintenir le second élément de mâchoire en position écartée de la seconde mâchoire lorsque le dispositif est en position ouverte.

Par ailleurs, on peut également prévoir un élément en forme de doigt formant saillie latéralement sur ledit second élément de mâchoire et s'engageant dans une encoche prévue sur le premier élément de mâchoire précité pour limiter le mouvement dudit second élément de mâchoire lorsque le dispositif est en position ouverte.

Selon encore une autre caractéristique du second mode de réalisation de l'invention, l'extrémité du premier élément de bras précité comprend un moyen de préhension en forme de bague, avantageusement de forme adaptée à la forme du doigt correspondant du praticien et l'extrémité du second élément de bras est simplement recourbée vers l'extérieur dudit dispositif pour simplement éviter le glissement du doigt vers l'extérieur de la pince.

Ainsi ce dispositif peut être aisément manipulé par le chirurgien qui a seulement deux doigts disposés dans les bagues de préhension du dispositif, le troisième doigt devant actionner le second élément de bras, étant simplement en appui sur celui-ci.

Les dispositifs d'application de l'invention permettent donc d'une part de prélever de manière sûre une pince hémostatique dans un dispositif de distribution, et d'autre part d'appliquer cette pince sur un conduit souple ou tel qu'un vaisseau sanguin, de réaliser un verrouillage positif de ladite pince hémostatique quelle que soit la grosseur du vaisseau ou de la masse de chair collée sur lui, et enfin de fermer la pince hémostatique pour obturer le vaisseau, ladite fermeture étant contrôlée directement par l'action de la pince sur le vaisseau.

Par ailleurs, les deux modes de réalisation d'un dispositif d'application de pinces hémostatiques de l'invention, qui comprennent chacun une rainure de centrage de la pince hémostatique formée sur la face interne de chaque mâchoire,comprennent avantageusement au moins dans la rainure de centrage de ladite seconde mâchoire, au moins un téton formant saillie dans ladite rainure et coopérant avec un évidement de la pince hémostatique.

Avantageusement la partie centrale de l'extrémité de la seconde mâchoire est recourbée vers la première mâchoire pour former un moyen de réception de la pince hémostatique, et pour permettre d'introduire cette pince hémostatique sans interférence avec le téton précité .

En outre, le premier élément de mâchoire précité comprend une extrémité formant un bec orienté vers ladite seconde mâchoire précitée et comprenant à son bord le plus externe une gorge centrale pour recevoir ladite extrémité recourbée de ladite seconde mâchoire et à son bord interne deux tétons latéraux coopérant avec des évidements latéraux de la pince hémostatique.

Selon encore une autre caractéristique, l'extrémité en forme de bec précitée du premier élément de mâchoire ainsi que les tétons précités sont fixés de manière amovible sur ledit dispositif d'application, par exemple, par vissage, rivetage ou analogue.

D'autres caractéristiques, détails et avantages de l'invention apparaîtront plus clairement à la lumière de la description explicative qui va suivre faite en référence aux dessins schématiques annexés, donnés uniquement à titre d'exemple illustrant deux modes de réalisation, et dans lesquels :

la figure 1 est une vue schématique avec arrachements partiaux, illustrant un premier mode de réalisation du dispositif d'application de pinces de l'invention, dans lequel est positionnée une pince hémostatique avant son application.

La figure 2 est une vue de dessus selon la flèche II de la figure 1.

La figure 3 est une vue selon la flèche III de la figure 1, illustrant la surface interne d'une mâchoire du dispositif d'application selon l'invention.

La figure 4 est une vue selon la flèche IV de la figure 1, illustrant la surface interne de l'autre mâchoire du dispositif d'application conforme à l'invention.

Les figures 5 à 7 sont des vues agrandies de l'extrémité du dispositif d'application de pinces selon le premier mode de réalisation de l'invention, illustrant les différentes positions des mâchoires et éléments de mâchoire lors de l'application d'une pince hémostatique sur un conduit souple.

La figure 8 est une vue schématique illustrant un second mode de réalisation du dispositif d'application de pinces de l'invention, en position ouverte ;

La figure 9 est une vue selon la flèche IX de la figure 8, illustrant la surface interne d'une mâchoire du second mode de réalisation du dispositif d'application ;

La figure 10 est une vue selon la flèche X de la figure 8, illustrant la surface interne de l'autre mâchoire de ce second mode de réalisation du dispositif d'application;

Les figures 11 à 13 sont des vues à échelle agrandie de l'extrémité des mâchoires du dispositif d'application de pinces de la figure 8., illustrant les différentes positions des mâchoires et éléments de mâchoires lors de l'application d'une pince hémostatique sur un conduit souple ; et

La figure 14 est une vue schématique, avec arrachements partiaux du second mode de réalisation du dispositif d'application illustrant la position du dispositif correspondant à la position des mâchoires de la figure 12.

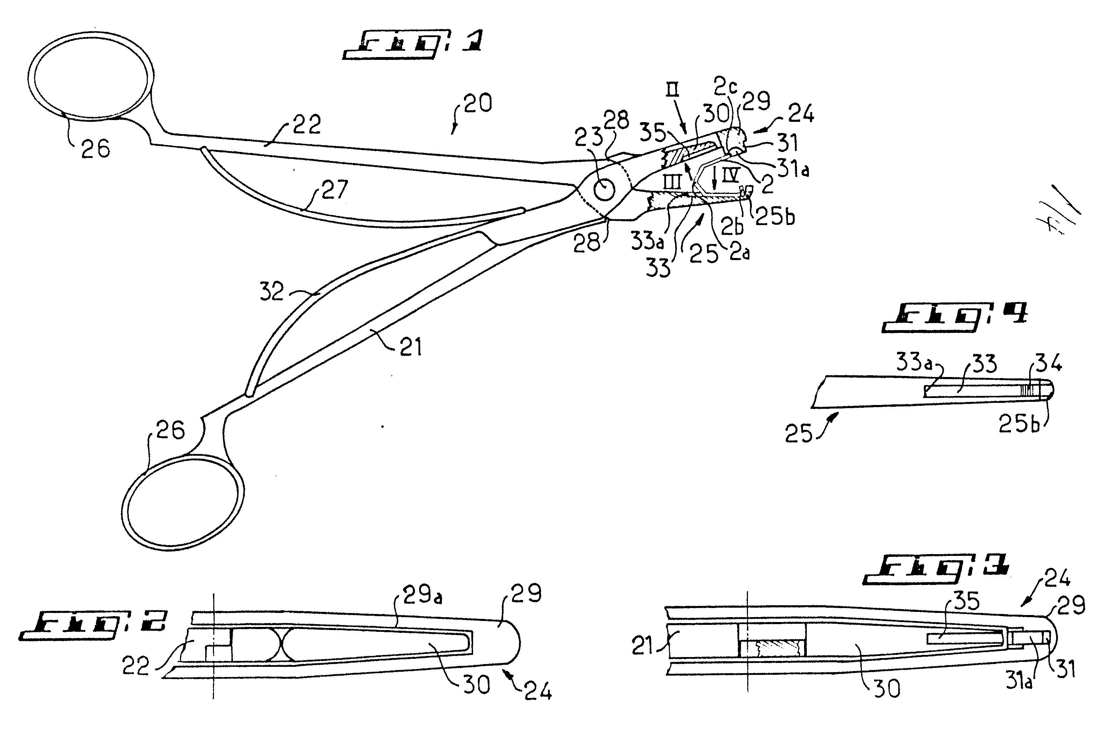

Avantageusement, chaque pince hémostatique 2 se compose d'une lame flexible présentant sensiblement en sa partie centrale une amorce de pliage 2a de façon à définir deux branches formant entre elles un angle relativement ouvert avec deux parties extrêmes 2c,2b libres. Une de ces parties extrêmes, par exemple 2b étant recourbée soit en forme de boucle, soit pour former un crochet, comme illustré dans les figures 1, 5 à 7.

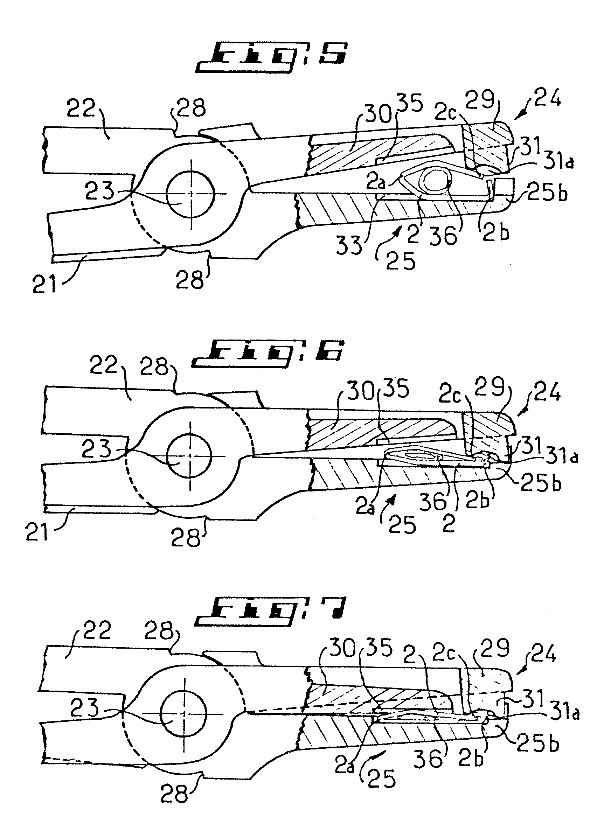

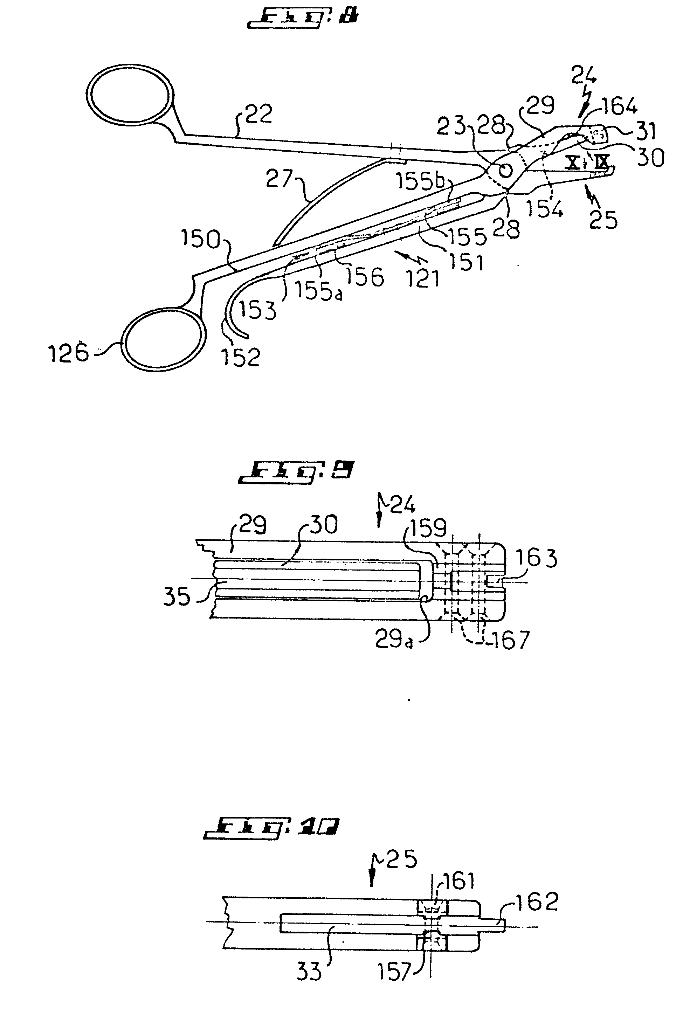

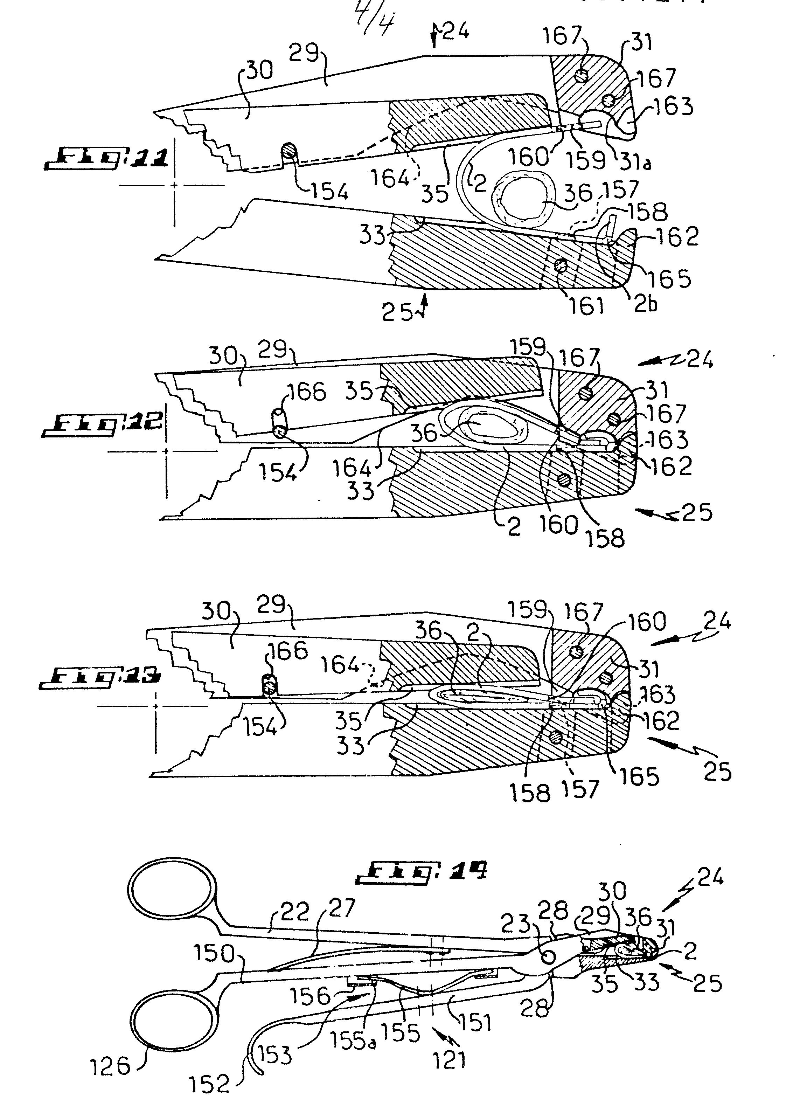

On décrira un premier mode de réalisation préféré du dispositif d'application de pinces hémostatiques 20 en se référant aux figures 1 à 7. Le dispositif d'application de pinces hémostatiques 20 est du type en forme de pince comprenant deux bras 21, 22 montés pivotants l'un par rapport à l'autre autour d'un axe de pivotement 23 pour déplacer des mâchoires 24, 25 entre lesquelles est disposée une pince hémostatique 2, notamment au moyen d'un dispositif de distribution ou support de pinces hémostatiques. Les bras 21, 22 présentent à leur extrémité libre des moyens de préhension 26 en forme de bague pour permettre l'insertion des doigts du chirurgien. Les deux bras 21, 22 sont maintenus en position écartée par un ressort 27 fixé par une de ses extrémités sur l'un des bras 22 et prenant appui par son autre extrémité sur l'autre bras 21. L'ouverture des bras, et donc des mâchoires est limitée par des butées 28. Dans le mode de réalisation préféré de l'invention, et comme illustré aux figures 5 à 6, ces butées 28 sont constituées par des épaulements prévus sur les bras 22, 21 au niveau de leur articulation autour de l'axe de pivotement 23. Il est bien entendu que d'autres modes de réalisation de ces butées peuvent être réalisés sans pour cela sortir du cadre de l'invention.

Conformément à l'invention, le dispositif d'application de pinces 20 comprend une mâchoire 24 comportant deux éléments de mâchoire 29, 30 agencés pour être appliqués successivement sur l'autre mâchoire 25.

Dans ce premier mode de réalisation illustré, le premier élément de mâchoire 29 comprend à son extrémité un élément 31 formant saillie vers l'autre mâchoire 25, venant de préférence de matière avec ledit élément de mâchoire 29, et présentant une gorge 31a à sa surface interne.

Avantageusement, ce premier élément 29 de mâchoire est relié à un bras déformable 32, monté pivotant autour de l'axe 23, et dont l'extrémité prend appui sur l'un des bras 21 par exemple.

Par ailleurs, cet élément de mâchoire 29 définit une ouverture centrale 29a dans laquelle est logé le second élément de mâchoire 30 qui est lui relié au bras 21. Dans le mode de réalisation préféré de l'invention, le bras 21 et le second élément de mâchoire 30 forment une seule pièce montée pivotante autour de l'axe de pivotement 23.

En se référant à la figure 4, la seconde mâchoire 25 présente sur sa face interne une rainure longitudinale 33 formant moyen de réception de la pince hémostatique 2. De plus, dans le cas où la pince hémostatique 2 utilisée présente une branche à extrémité recourbée 2b, la mâchoire 25 a une extrémité recourbée 25b pour former moyen de réception, et pour guider et maintenir au moins une partie de l'extrémité recourbée 2b de la pince hémostatique 2.

Avantageusement, la rainure 33 peut comporter au moins une saillie transversale 34, de forme de préférence en dent de scie, coopérant par exemple avec des saillies transversales prévues sur la surface externe au voisinage des extrémités, des branches de la pince hémostatique 2.

En outre, la gorge longitudinale 33 se termine par un épaulement 33a formant butée pour maintenir la pince hémostatique 2 en position correcte notamment pendant la fermeture des mâchoires 24, 25. Cette butée 33a peut être également constituée par un élément ayant par exemple des propriétés élastiques formant saillie à la surface de la mâchoire 25, l'élément de mâchoire 30 comportant alors un évidement de réception de ladite butée en position fermée des mâchoires 24, 25.

De plus, comme illustré à la figure 3, le second élément comprend également une rainure longitudinale 35 permettant de maintenir la pince hémostatique 2 en position correcte lors de la fermeture des mâchoires 24 et 25.

En se référant plus particulièrement aux figures 5 à 7, on décrira le fonctionnement du premier mode de réalisation du dispositif d'application 20 de la présente invention.

Après prise d'une pince hémostatique 2, par exemple, par insertion de l'extrémité des mâchoires 24, 25 dans un dispositif de distribution ou de support de pinces hémostatiques, la pince hémostatique 2 est disposée entre les mâchoires du dispositif d'application de pinces 20 dans la position représentée à la figure 1. Ainsi, une branche de la pince hémostatique 2 est en contact avec la mâchoire 25, l'extrémité de l'autre branche étant en contact avec l'élément déformant 31, c'est-à-dire l'extrémité du premier élément de mâchoire 29.

Toutefois, et sans pour cela sortir du cadre de l'invention, il est bien entendu possible que le second élément de mâchoire 30 soit en contact avec une branche de la pince hémostatique 2 pour avoir ainsi un maintien de la pince hémostatique 2 amélioré.

Le chirurgien positionne alors, par l'intermédiaire des bras 22, 21 la pince hémostatique 2 autour d'un conduit souple tel qu'un vaisseau sanguin 36, puis par rapprochement des deux bras 22, 21 c'est-à-dire contre la force du ressort 27, le chirurgien applique le premier élément de mâchoire 9, sur la mâchoire 25 et ainsi effectue un rapprochement des extrémités des deux branches de la pince hémostatique 2 pour former une boucle fermée comme illustré à la figure 6. Au cours de ce rapprochement, l'extrémité 2b recourbée de la pince vient en contact avec le fond de la gorge 31a qui a une forme courbe convexe pour replier l'extrémité 2b au-dessus de l'autre extrémité
droite de la pince hémostatique 2.

Ainsi, dans la position illustrée à la figure 6, la
pince hémostatique 2 a ses extrémités verrouillées
positivement, toutefois le vaisseau sanguin 36 n'est
pas obturé.

A ce moment là, par actionnement des bras 21, 22
contre la force de ressort 32 du bras déformable relié au premier élément de mâchoire 29, le second
élément de mâchoire 30 est appliqué sur la mâchoire
25 pour écraser la boucle formée et ainsi obturer le
vaisseau sanguin 36. Toutefois, cet écrasement de la
pince hémostatique 2 est seulement limité par l'épaisseur de la paroi du vaisseau sanguin 36 et en conséquence le chirurgien peut contrôler de manière effective l'obturation de ce vaisseau.

Comme illustré à la figure 7, on voit clairement que
lors de l'écrasement de la pince hémostatique 2, la
branche droite coulisse à l'intérieur de l'extrémité
recourbée de l'autre branche, permettant ainsi d'améliorer le verrouillage de la pince hémostatique.

En conséquence, le premier mode de réalisation du
dispositif d'application de pinces hémostatiques 20
de l'invention permet dans un premier temps de réaliser un verrouillage positif de la pince hémostatique  par chevauchement de ses deux extrémités en
formant une boucle fermée. Il est aisé de comprendre
que ce verrouillage est réalisé quelle que soit la grosseur du vaisseau sanguin 36, ou de la masse de chair qui est accrochée à sa paroi. Ensuite, le chirurgien peut par actionnement sur les bras 21, 22 du dispositif d'application de pinces 20, écraser la boucle pour obturer le vaisseau 36, cette obturation étant contrôlée uniquement par la résistance à l'écrasement du vaisseau 36.

On décrira maintenant, en se référant aux figures 8 à 14, un second mode de réalisation du dispositif d'application de pinces hémostatiques conforme à l'invention.

En se reportant à la figure 8, le dispositif d'application de pinces hémostatiques est du type en forme de pince comprenant deux bras 22, 121 montés pivotant l'un par rapport à l'autre autour d'un axe de pivotement 23 pour déplacer les mâchoires 24, 25 entre lesquelles est disposée une pince hémostatique 2. Cette pince hémostatique est par exemple, disposée entre les deux mâchoires 24 et 25, au moyen d'un dispositif de distribution de pinces.

Les deux bras 22, 121 sont maintenus en position écartée par un ressort 27 fixé par l'une de ses extrémités sur l'un des bras 22 et prenant appui par son autre extrémité sur l'autre bras 121.

Par ailleurs, l'ouverture des bras et donc des mâchoires est limitée par des butées 28. La mâchoire 24 comporte deux éléments de mâchoires 29, 30 agencés pour être appliqués successivement sur l'autre mâchoire 25. Le premier élément de mâchoire 29 comprend à son extrémité un élément 31 formant saillie vers l'autre mâchoire 25, pour définir ainsi un bec, et présente une ouverture centrale 29a dans laquelle est logé le second élément de mâchoire 30. De plus, le bec 31 présente à sa surface interne une gorge 31a dont le fond à une configuration permettant de recourber vers l'intérieur et fermer l'extrémité d'une pince hémostatique comme cela est clairement illustré dans les figures 11 à 13, et décrit ci-dessus.

Par ailleurs, les surfaces internes de la seconde mâchoire 25 et du second élément de mâchoire 30 comprennent respectivement une rainure centrale 33, 35 pour le centrage de la pince hémostatique 2.

Toutes ces dispositions ont déjà été décrites pour le premier mode de réalisation du dispositif d'application de pinces hémostatiques.

Dans ce second mode de réalisation, le bras 121 du dispositif d'application comprend un premier élément de bras 150 comprenant à son extrémité un moyen de préhension en forme de bague 126 et étant relié au premier élément de mâchoire 29 de la première mâchoire 24.

Avantageusement, le premier élément de mâchoire 29 et l'élément de bras 150 viennent de matière, l'ensemble étant monté en rotation autour de l'axe 23.

Par ailleurs, le bras 121 comprend un second élément de bras 151 relié au second élément de mâchoire 30 et monté également en rotation autour de l'axe 23.

Ce second élément de bras 151 a, avantageusement, une extrémité 152 recourbée vers l'extérieur et est légèrement écartée du bras 150 en position ouverte du dispositif d'application.

L'élément de mâchoire 30 et le bras 151 sont avantageusement d'une seule pièce.

De plus, pour éviter que l'élément de bras 151 s'écarte de l'élément de bras 150, quand le dispositif d'application est en position ouverte et donc pour limiter l'ouverture de l'élément de mâchoire 30, on prévoit un élément formant butée qui est, par exemple, soit un élément 153 formant ressort de rappel entre les deux branches, soit un doigt 154 fixé latéralement sur la mâchoire 30 et venant en engagement dans une encoche 166 de la mâchoire 29, comme illustré clairement aux figures 11 à 13. Il est également possible de réaliser un dispositif d'application comprenant ces deux dispositifs 153, 154.

Dans le second mode de réalisation représenté, l'élément formant ressort de rappel 153 est constitué par une lame de ressort 155 fixée par sa partie centrale à l'élément de bras 151 et dont les extrémités 155a, 155b sont montées coulissantes dans des logements 156 prévus sur l'élément de bras 150. La force de rappel du ressort 155 doit être relativement faible et avantageusement inférieure à la force nécessaire pour déformer la pince hémostatique 2, et nettement inférieure à la résistance à la déformation du conduit souple ou vaisseau sanguin 36.

Par ailleurs, au moins un téton 157, de préférence deux tétons, sont montés en opposition dans la mâchoire 25 de chaque côté de la rainure centrale 33. Ces tétons viennent en engagement dans des évidements latéraux 158 réalisés dans la pince hémostatique 2 pour former butée d'arrêt et d'immobilisation de la pince hémostatique au cours de son application sur un conduit souple. Dans ce second mode de réalisation préféré de l'invention, on prévoit également deux tétons 159 sensiblement identiques sur le bord interne du bec 31 du premier élément de mâchoire 29 qui viennent en engagement dans des évidements latéraux conjugués 160 de la pince hémostatique 2.

De préférence, ces tétons 157, 159 sont montés de manière amovible sur la mâchoire 25 ou le bec 31 par vissage ou rivetage illustré schématiquement en 161.

Bien entendu, le ou les tétons 157 peuvent être prévus dans la partie centrale de la rainure 33, pour coopérer avec un évidement 160 conjugué de la pince hémostatique, sans pour cela sortir du cadre de l'invention.

Avantageusement, pour permettre une insertion aisée de la pince 2 entre les mâchoires 25, 24 du dispositif d'application, sans interférence avec les tétons 159, 157, uniquement la partie centrale 162 de l'extrémité de la mâchoire 25 est recourbée vers le haut pour former un moyen de réception de la pince hémostatique et pour maintenir celle-ci en position surélevée par rapport aux tétons 157 lors de l'insertion de la pince 2 dans le dispositif d'application.

Pour permettre l'application de l'élément de mâchoire 29 sur la mâchoire 25, le bord externe du bec 31 présente une gorge centrale 163 de forme conjuguée à la partie centrale recourbée 162 de la mâchoire 25.

La largeur de cette gorge centrale 163 est plus petite que la largeur de la branche de la pince hémostatique 2, pour maintenir celle-ci en position surélevée par rapport aux tétons 159 pendant l'insertion de la pince 2 dans le dispositif d'application, et avantageusement provoquer une déformation symétrique de la pince 2.

Avantageusement, le bec 31 est monté de manière amovible sur l'élément de mâchoire 29 pour ainsi permettre son remplacement lorsqu'il est usé, par exemple par des rivets 167.

Enfin, l'élément de mâchoire 29 comprend immédiatement en amont du bec 31, un évidement 164 pour permettre le passage du conduit souple 36.

En se référant plus particulièrement aux figures 11 à 14, on décrira le fonctionnement du second mode de réalisation du dispositif d'application.

Après prise d'une pince hémostatique 2, par exemple par insertion de l'extrémité des mâchoires 24, 25 dans le logement d'un dispositif de distribution de pinces hémostatiques, la pince hémostatique 2 est disposée entre les mâchoires du dispositif d'application de pinces, les tétons 157, 159 étant engagés dans les évidements 158, 160 conjugués.

Ainsi, la pince hémostatique 2 est centrée et maintenue en position longitudinale dans le dispositif d'application.

Par ailleurs, le second élément de mâchoire 30 est généralement en contact avec une branche de la pince hémostatique 2 pour ainsi encore améliorer son maintien.

Le chirurgien positionne alors, par l'intermédiaire des bras 22 et 150 la pince hémostatique 2 autour d'un conduit souple tel qu'un vaisseau sanguin 36, puis par rapprochement des deux bras 22, 150, c'est-à-dire contre la force du ressort 27, le chirurgien applique le premier élément de mâchoire 29 sur la mâchoire 25 et ainsi effectue un rapprochement des extrémités des deux branches de la pince hémostatique 2 pour former une boucle fermée comme illustrée à la figure 12. Au cours de ce rapprochement, l'extrémité 2b recourbée de la pince vient en contact avec le fond de la gorge 31a du bec 31 qui a une forme courbe convexe pour replier l'extrémité 2b au dessus de l'autre extrémité droite de la pince hémostatique 2.

Ainsi, dans la position illustrée aux figures 12, 13 la pince hémostatique 2 a ses extrémités verrouillées positivement, toutefois le vaisseau sanguin 36 n'est pas obturé.

Comme illustré à la figure 14, dans cette position, les bras 22 et 150 sont en position rapprochée pour fermer la pince constituée par la mâchoire 25 et le premier élément de mâchoire 29, l'élément de bras 151 étant en position plus ou moins écartée vis-à-vis de l'élément de bras 150. De ce fait, pour obturer le vaisseau sanguin 36, par écrasement de la boucle de la pince hémostatique 2, et donc pour fermer l'élément de mâchoire 30 sur la mâchoire 25, il suffit de rapprocher le second élément de bras 151 contre l'élément de bras 150. Ce rapprochement est effectué par application d'une force permettant de vaincre sensiblement uniquement la force de résistance à l'écrasement du vaisseau sanguin 36, la force de résistance à la déformation de la pince 2 étant faible devant celle du vaisseau sanguin.

En conséquence, ce dispositif d'application permet au chirurgien de contrôler la force appliquée pour l'obturation d'un vaisseau sanguin en fonction de la résistance de ce vaisseau à l'écrasement.

Une fois le serrage effectué, par relâchement des efforts, le ressort 27 provoque une ouverture du dispositif d'application.

La présente invention propose donc un dispositif d'application de pinces hémostatiques permettant d'obturer un vaisseau sanguin par application d'une force correspondant sensiblement à la résistance à l'écrasement du vaisseau. Par ailleurs, la pince hémostatique est maintenue en position entre les deux mâchoires par d'une part des rainures centrales et d'autre part des tétons de butées d'arrêt permettant d'éviter des déplacements longitudinaux vers l'arrière ou vers l'avant des mâchoires lors de l'écrasement de cette pince. Ces tétons peuvent bien entendu être également disposés dans les deux modes de réalisation du dispositif d'application de pinces hémostatiques.

Par ailleurs, pour permettre un recourbement aisé de l'extrémité courbée de la pince hémostatique 2, et un verrouillage plus facile de cette pince, des évidements latéraux 165 sont prévus au niveau de la courbure de l'extrémité 2b de la pince hémostatique 2.

Il est bien entendu possible, sans pour cela sortir du cadre de l'invention, de prévoir des modes de réalisation différents pour les butées de limitation d'ouverture 28, 154, 153, et notamment des butées à position réglable.

Bien entendu, l'invention n'est nullement limitée aux modes de réalisation du dispositif d'application de pinces hémostatiques décrits et représentés qui n'ont été donnés qu'à titre d'exemple. C'est ainsi que les éléments de mâchoire 29, 30 constituant la mâchoire 24 du dispositif d'application 20 peuvent être rendues solidaires en pivotement, des bras 21 et 22 du dispositif par tout moyen technique équivalent tel que butée, came, ou analogues. La forme de l'extrémité des mâchoires 24 et 25, est adaptée au profil de la pince hémostatique utilisée.

Revendications

1.- Procédé d'application de pinces hémostatiques consistant par exemple à retirer une pince hémostatique notamment d'un dispositif de distribution de pinces, au moyen d'un dispositif d'application de pinces, puis loger un conduit souple à obturer tel qu'un vaisseau sanguin entre les deux branches de la pince hémostatique et à obturer ledit conduit par fermeture de ladite pince, caractérisé en ce que pour fermer ladite pince on réalise successivement un rapprochement des extrémités des branches de la pince pour former une boucle, un recouvrement desdites extrémités pour verrouiller ladite pince, puis un écrasement de la boucle ainsi formée jusqu'à obturation dudit conduit souple.

2.- Dispositif d'application de pinces hémostatiques pour la mise en oeuvre du procédé selon la revendication 1, ledit dispositif étant en forme de pince comprenant deux bras reliés de manière pivotante et actionnant deux mâchoires, lesdits bras comportant à leur extrémité libre des moyens de préhension généralement en forme de bague, caractérisé en ce que l'une des mâchoires (24) comprend deux éléments de mâchoires (29,30) agencés pour être appliqués successivement sur l'autre mâchoire (25), un premier élément (29) agencé pour former une boucle avec recouvrement des extrémités de la pince hémostatique (2), et un second élément pour écraser la boucle ainsi formée jusqu'à obturation complète du conduit souple (36).

3.- Dispositif selon la revendication 2, caractérisé en ce que le premier élément de mâchoire (29) est relié à un organe (32) élastiquement déformable,

22

monté de manière pivotante avec les deux bras (21,22) précités des dispositifs (20), le pivotement et la déformation desdits organes (32) étant commandées par lesdits bras (22,21).

4.- Dispositif selon la revendication 2 ou 3, caractérisé en ce que le second élément de mâchoire (30) est disposé dans une ouverture centrale (29a) dudit premier élément de mâchoire (29), ledit second élément de mâchoire (30) étant relié au moins temporairement à l'un des bras (21) dudit dispositif (20).

5.- Dispositif selon la revendication 4, caractérisé en ce que le second élément de mâchoire (30) vient de matière avec l'un des bras (21) dudit dispositif (20).

6.- Dispositif selon l'une des revendications 2 à 5, caractérisé en ce qu'au moins une mâchoire (25,24) comporte à sa surface interne une rainure longitudinale (33,35) formant moyen de réception de la pince hémostatique (2).

7.- Dispositif selon la revendication 6, caractérisé en ce qu'au moins une partie de la rainure (33,35) précitée présente au moins une saillie transversale (34), avantageusement en forme de dent de scie.

8.- Dispositif selon l'une des revendications 6 ou 7, caractérisé en ce qu'au moins un des moyens de réception précité ou rainures (33,35) comporte une butée (33a) espacée de l'extrémité d'une mâchoire (24,25) du dispositif (20).

9.- Dispositif selon l'une des revendications 2 à 8, pour l'application de pinces hémostatiques comportant une branche à extrémité recourbée, caractérisé en ce que la mâchoire (25) comprend une extrémité (25b) recourbée formant moyen de réception de ladite pince et susceptible de retenir et guider au moins une partie de ladite extrémité recourbée de la pince hémostatique (2), l'extrémité du premier élément de mâchoire précité (29) comprenant un élément déformant susceptible d'agir sur ladite extrémité (2b) recourbée lors d'un mouvement de fermeture des mâchoires (24,25).

10.- Dispositif selon la revendication 9, caractérisé en ce que l'élément déformant précité (31) comprend à sa surface interne une gorge (31a) dont le fond définit une courbe convexe pour guider et plier l'extrémité recourbée (2b) de la pince hémostatique, au-dessus de l'autre extrémité (2c) de ladite pince.

11.- Dispositif selon la revendication 9 ou 10, caractérisé en ce que l'élément déformant précité (31) vient de matière avec ledit premier élément de mâchoire (29).

12.- Dispositif d'application de pinces hémostatiques, selon l'une des revendications 2, 4, 6 à 8 caractérisé en ce que le premier bras (121) précité comprend un premier élément de bras (150) relié au premier élément précité de mâchoire (29) et un second élément de bras (151) relié au second élément de mâchoire (30) précité.

13.- Dispositif selon la revendication 12, caractérisé en ce qu'un élément (153) formant ressort de rappel est disposé entre les premier (150) et second (151) éléments de bras précité, pour maintenir ouvert le second élément de mâchoire (30) quand le dispositif est en position ouverte.

14.- Dispositif selon la revendication 12 ou 13, caractérisé en ce que l'extrémité du premier élément de bras (150) précité comprend un moyen de préhension (126) en forme de bague, tandis que l'extrémité (152) du second élément de bras (151) précité est recourbée vers l'extérieur dudit dispositif.

15.- Dispositif selon l'une des revendications précédentes, caractérisé en ce qu'il comprend au moins un élément formant butée pour limiter l'ouverture du second élément de mâchoire (30) par rapport audit premier élément de mâchoire (29).

16.- Dispositif selon la revendication 15, caractérisé en ce que l'élément formant butée précité est un doigt (154) formant saillie sur une face latérale du second élément de mâchoire (30) et s'engageant dans une encoche (166) du premier élément de mâchoire (29) précité.

17.- Dispositif selon l'une des revendications 1 à 9 et 12 à 16, caractérisé en ce qu'au moins un téton (157) formant saillie est prévu dans une partie de la rainure de centrage précitée (33) de la seconde mâchoire (25) pour coopérer avec au moins un évidement (158) conjugué de la pince hémostatique (2).

18.- Dispositif selon la revendication 17, caractérisé en ce que la partie centrale (162) de l'extrémité de la seconde mâchoire (25) précitée est recourbée vers la première mâchoire (24) pour former un moyen de réception de la pince hémostatique (2).

19.- Dispositif selon l'une des revendications 12 à 18, caractérisé en ce que le premier élément de mâchoire (29) précité comprend une extrémité (31) formant bec, orientée vers la seconde mâchoire (25) précitée et comprenant une gorge centrale (31a) et à son bord externe, une gorge centrale (163) de forme conjuguée à l'extrémité recourbée (162) de la seconde mâchoire (25) précitée et de largeur sensiblement inférieure à la largeur de la pince hémostatique (2), et à son bord interne, au moins un téton (159) latéral s'engageant dans des évidements latéraux conjugués de la pince hémostatique (2).

20.- Dispositif selon la revendication 19, caractérisé en ce que l'extrémité en forme de bec précitée (31) est fixée de manière amovible sur le premier élément de mâchoire (29) précité.

21.- Dispositif selon la revendication 17 ou 20, caractérisé en ce que les tétons (157,159) sont fixés de manière amovible respectivement sur la seconde mâchoire (25) et sur le premier élément de mâchoire (29).

22.- Pince hémostatique appliquée par le dispositif d'application selon l'une des revendications 17 à 19 du type comprenant une lame métallique repliée pour former deux branches, caractériséę en ce qu'elle comprend au moins un évidement (158,160) sur une de ses branches pour coopérer avec chaque téton (157,159) précité prévu sur les mâchoires du dispositif d'application.

23.- Pince selon la revendication 22, dont une de ses branches a une extrémité recourbée vers l'autre branche, caractérisée en ce que des évidements (165) sont prévus au niveau de la courbure de ladite extrémité (2b) recourbée.

_Fig. 1_

II 2c
23 28 35 30 29 24
20
22
27
31
31a
III IV 2
28 33a 2b 25b
33 25 2a

32

21

26

26

_Fig. 4_

33a 33 34
25
25b

_Fig. 2_

29a 29

22 30 24

_Fig. 3_

24
35 29
21
30 31a 31

0077277

Fig. 5

Fig. 6

Fig. 7

3/4

0077277

Fig. 8

Fig. 9

Fig. 10

FIG. 11

FIG. 12

FIG. 13

FIG. 14

Office européen
des brevets

## RAPPORT DE RECHERCHE EUROPEENNE

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | A 61 B 17/12 |
| Y | US-A-3 270 745 (WOOD) <br> * colonne 4, lignes 16-63; figures 1,16-24 * | 1 | |
| A | | 6,8,17 ,22 | |
| Y | --- <br> US-A-3 780 416 (RIDER) <br> * colonne 3, lignes 1-9; colonne 5, lignes 17-38; figures 1,2,4,7,8 * | 1 | |
| A | | 9,10 | |
| A | --- <br> FR-E- 3 926 (MICHEL) <br><br> * page 1, ligne 38 - page 2, ligne 48; figures 1-5 * | 2,3,12 ,13 | |
| A | --- <br> US-A-2 626 608 (GARLAND) <br> * colonne 2, ligne 39 - colonne 4, ligne 43; figures 1-5 * | 1,9 | |
| A | --- <br> FR-A-1 481 438 (McDERMOTT) <br><br> * page 4, colonne de gauche, ligne 52 - colonne de droite, ligne 34; figures 1-5 * | 1,6,8, 9,11, 17,18 | |
| | --- -/- | | |

**DOMAINES TECHNIQUES RECHERCHES (Int. Cl. ³)**

A 61 B

Le présent rapport de recherche a été établi pour toutes les revendications

| Lieu de la recherche | Date d'achèvement de la recherche | Examinateur |
|---|---|---|
| LA HAYE | 19-01-1983 | BARTLETT S.C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503. 03.82

Office européen
des brevets

**RAPPORT DE RECHERCHE EUROPEENNE**

Numéro de la demande

EP 82 40 1886

| | **DOCUMENTS CONSIDERES COMME PERTINENTS** | | Page 2 |
|---|---|---|---|

| Catégorie | Citation du document avec indication, en cas de besoin, des parties pertinentes | Revendication concernée | CLASSEMENT DE LA DEMANDE (Int. Cl. ³) |
|---|---|---|---|
| A | FR-A-2 462 646 (HROUDA)<br><br>* page 3, ligne 22 - page 5, ligne 14; figures 1-4 * | 1,6,8, 9,11, 17,18 | |
| A | US-A-3 735 762 (BRYAN)<br><br>* colonne 6, lignes 20-28; colonne 12, lignes 5-23; figures 1,15,20-25 * | 1,6,9, 10 | |
| A | US-A-2 123 890 (GOSSRAU)<br>* colonne 2, lignes 1-39; figures 1,2 * | 1 | |
| A | US-A-3 175 556 (WOOD)<br>* colonne 2, ligne 70 - colonne 3, ligne 71; colonne 4, ligne 36 - colonne 5, ligne 3; figures 1-6 * | 12-16 | DOMAINES TECHNIQUES RECHERCHES (Int. Cl. ³) |
| A | FR-A-2 476 478 (ETHICON)<br>* page 8, ligne 23 - page 9, ligne 26; page 11, lignes 25-31; figures 3,5,6 * | 17,22 | |
| A | GB-A-2 054 027 (ETHICON)<br>* page 2, lignes 101-114; page 3, lignes 70-74; figures 3,5,6 * | 17,22 | |

| Le présent rapport de recherche a été établi pour toutes les revendications | | |
|---|---|---|
| Lieu de la recherche<br>LA HAYE | Date d'achèvement de la recherche<br>19-01-1983 | Examinateur<br>BARTLETT S.C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82